# EUROPEAN PATENT APPLICATION

(11) **EP 4 541 798 A1**
(43) Date of publication of application: **23.04.2025**
(21) Application number: 24767195.1
(22) Date of filing: 06.03.2024
(51) Int. Cl.: C07F 1/08, C07C 17/266, C07C 19/10, C07C 21/18, C07D 471/04

(54) **PRODUCTION METHOD FOR FLUOROALKYL AROMATIC COMPOUND**

(30) Priority: 07.03.2023 JP 2023034958
(71) Applicant: DAIKIN INDUSTRIES, LTD., Osaka-shi, Osaka 530-0001 (JP); OSAKA UNIVERSITY, Suita-shi Osaka 565-0871 (JP)
(72) Inventor: HOSHIYA, Naoyuki, Osaka-Shi, Osaka 530-0001 (JP); YAMAUCHI, Akiyoshi, Osaka-Shi, Osaka 530-0001 (JP); ADACHI, Kenji, Osaka-Shi, Osaka 530-0001 (JP); NAMIKAWA, Takashi, Osaka-Shi, Osaka 530-0001 (JP); OGOSHI, Sensuke, Suita-shi, Osaka 565-0871 (JP); DOI, Ryohei, Suita-shi, Osaka 565-0871 (JP)
(74) Representative: Hoffmann Eitle
(86) International application number: PCT/JP2024/008622
(87) International publication number: WO 2024/185829

(57) **Abstract**

Provided is a useful method for producing a fluoroalkyl aromatic compound and a compound obtained by the production method. The production method is a method for producing a compound represented by formula (4), the method comprising step C of reacting, in the presence of a reducing agent, a copper compound, and a ligand, a compound represented by formula (1) and a compound represented by formula (3):

RF-X¹ (1),

Ar-X2 (3),

Ar-R^{F} (4),

wherein R^{F} is a fluoroalkyl group optionally having one or more substituents, X¹ is Cl, Br, or I, X² is Cl, Br, or I, and Ar is an aryl group optionally having one or more substituents or a heteroaryl group optionally having one or more substituents.

## Description

### Technical Field

The present disclosure relates to, for example, a method for producing a fluoroalkyl aromatic compound.

### Background Art

Fluoroalkyl aromatic compounds are useful compounds as agrochemicals, pharmaceuticals, liquid crystal materials, and the like. Known methods for producing such compounds include, for example, a method for producing a fluoroalkyl aromatic compound by reacting a fluoroalkyl iodide, such as C₅F₁₁I or C₆F₁₃I, an aryl iodide, such as iodobenzene, and copper powder in a high-boiling point solvent, such as N,N-dimethylformamide (DMF) or dimethyl sulfoxide (DMSO) (PTL 1, PTL 2, and NPL 1).

### Citation List

### Patent Literature

PTL 1: US Patent No. 3,408,411
PTL 2: US Patent Publication No. 2016/152636

### Non-patent Literature

NPL 1: D. Wiedenfeld et al., Journal of Fluorine Chemistry, 104 (2000), 303-306

### Summary of Invention

### Technical Problem

The method of PTL 1 requires high-temperature conditions, and in addition, the method of PTL 1 has room for improvement in terms of yield. Additionally, the methods of PTL 1, PTL 2, and NPL 1 all use a high-boiling point solvent and thus require complicated operations multiple times, such as extraction with water, vacuum distillation, and crystallization, in order to collect the product from the high-boiling point solvent.

An object of the present disclosure includes providing a useful method for producing a fluoroalkyl aromatic compound, and a compound obtained by the production method.

### Solution to Problem

The present disclosure encompasses the following embodiments.
Item 1. A method for producing a compound represented by formula (2):

   M-R^{F} (2),

   wherein M is Cu having a ligand, and
   R^{F} is a fluoroalkyl group optionally having one or more substituents;
      the method comprising:
      step A of reacting, in the presence of a reducing agent, a compound represented by formula (1):

         R^{F}-X¹ (1),
   wherein R^{F} is as defined above, and
   X¹ is Cl, Br, or I;
   a copper compound, and a ligand.
Item 2. A method for producing a compound represented by formula (4):

   Ar-R^{F} (4),

   wherein Ar is an aryl group optionally having one or more substituents or a heteroaryl group optionally having one or more substituents, and
   R^{F} is a fluoroalkyl group optionally having one or more substituents;
      the method comprising:
      step A of reacting, in the presence of a reducing agent, a compound represented by formula (1):

         R^{F}-X¹ (1),

         wherein R^{F} is as defined above, and
         X¹ is Cl, Br, or I;
         a copper compound, and a ligand; and
      step B of reacting a reaction product of step A and a compound represented by formula (3):

         Ar-X² (3),
         wherein Ar is as defined above, and
         X² is Cl, Br, or I.
Item 3. A method for producing a compound represented by formula (4):

   Ar-R^{F} (4),
   wherein Ar is an aryl group optionally having one or more substituents or a heteroaryl group optionally having one or more substituents, and
   R^{F} is a fluoroalkyl group optionally having one or more substituents;
      the method comprising:
      step C of reacting, in the presence of a reducing agent, a copper compound, and a ligand, a compound represented by formula (1):

         R^{F}-X¹ (1),

         wherein R^{F} is as defined above, and
         X¹ is Cl, Br, or I;
         and a compound represented by formula (3):

            Ar-X² (3),

            wherein Ar is as defined above, and
            X² is Cl, Br, or I.
Item 4. The production method according to any one of Items 1 to 3, wherein the reducing agent is a compound containing boron and/or silicon.
Item 5. The production method according to any one of Items 1 to 4, wherein the reducing agent is at least one member selected from the group consisting of a diboron compound, a silylborane compound, and a disilane compound.
Item 6. The production method according to Item 5,
   wherein the diboron compound is a compound represented by formula (A): 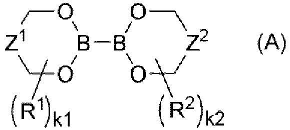
      wherein Z¹ and Z² are each independently a single bond, a double bond, or CH₂,
      R¹ and R² are each independently an alkyl group,
      two adjacent R¹s, when present, are optionally bonded together to form a ring,
      two adjacent R²s, when present, are optionally bonded together to form a ring, and
      k1 and k2 are each independently an integer of 0 or 1 or more;
   the silylborane compound is a compound represented by formula (B): 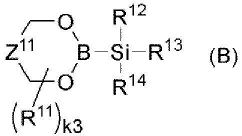
      wherein Z¹¹ is a single bond, a double bond, or CH₂,
      R¹¹ is an alkyl group,
      two adjacent R¹¹s, when present, are optionally bonded together to form a ring,
      R¹², R¹³, and R¹⁴ are each independently an alkyl group or an aryl group, and
      k3 is an integer of 0 or 1 or more; and
   the disilane compound is a compound represented by formula (C): 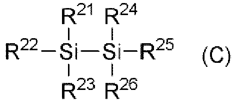 wherein R²¹, R²², R²³, R²⁴, R²⁵, and R²⁶ are each independently H, halogen, an alkyl group, an alkoxy group, or an aryl group.
Item 7. The production method according to any one of Items 1 to 6, which is performed in an organic solvent.
Item 8. The production method according to Item 7, wherein the organic solvent has a boiling point of 150°C or lower under ordinary pressure.
Item 9. The production method according to Item 7 or 8, wherein the organic solvent is at least one member selected from the group consisting of ether solvents and aromatic hydrocarbon solvents.
Item 10. The production method according to any one of Items 1 to 9, wherein the copper compound is a monovalent copper compound.
Item 11. The production method according to any one of Items 1 to 10, wherein the copper compound is an alkoxide, aryloxide, thioalkoxide, or thioaryloxide of Cu.
Item 12. The production method according to any one of Items 1 to 11, wherein the ligand is at least one member selected from the group consisting of pyridine-containing ligands and phosphine ligands.
Item 13. The production method according to any one of Items 2 to 12, wherein step B or step C is performed at 50°C or lower.
Item 14. The production method according to any one of Items 2 to 13, wherein Ar is an aryl group optionally having one or more substituents.
Item 15. The production method according to any one of Items 2 to 14, wherein Ar is an aryl group having one or more fluorine atoms or fluoroalkyl groups.
Item 16. A compound represented by formula (4A): 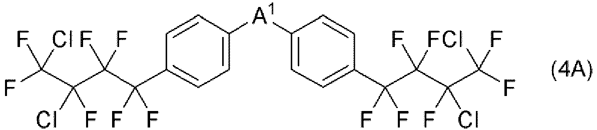 wherein A¹ is a single bond, -O-, -S-, an alkylene group optionally having one or more substituents, a cycloalkylene group optionally having one or more substituents, or an arylene group optionally having one or more substituents.
Item 17. A compound represented by formula (5A): 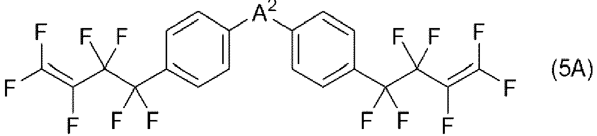 wherein A² is a single bond, -O-, -S-, an alkylene group optionally having one or more substituents, a cycloalkylene group optionally having one or more substituents, or an arylene group optionally having one or more substituents.
Item 18. A compound represented by formula (5A): 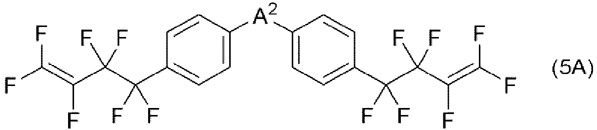 wherein A² is a single bond, -O-, -S-, an alkylene group optionally having one or more substituents, a cycloalkylene group optionally having one or more substituents, or an arylene group optionally having one or more substituents; and wherein the content of each of Al, Cr, Cu, Fe, Ni, and Zn is 1000 ppm or less.
Item 19. A compound represented by formula (5A): 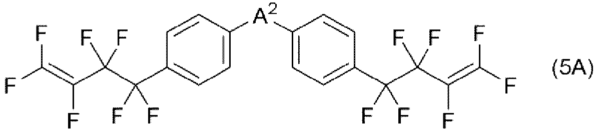 wherein A² is a single bond, -O-, -S-, an alkylene group optionally having one or more substituents, a cycloalkylene group optionally having one or more substituents, or an arylene group optionally having one or more substituents; and wherein the total content of Al, Cr, Cu, Fe, Ni, and Zn is 6000 ppm or less.
Item 20. A compound represented by formula (5): 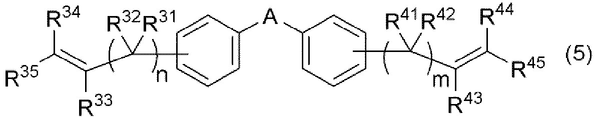
   wherein A is a single bond, -O-, -S-, an alkylene group optionally having one or more substituents, a cycloalkylene group optionally having one or more substituents, or an arylene group optionally having one or more substituents,
   R³¹, R³², R²³, R³⁴, R³⁵, R⁴¹, R⁴², R⁴³, R⁴⁴, and R⁴⁵ are each independently fluorine, an alkyl group, or a fluoroalkyl group, and
   n and m are each independently 0, 1, or 2; and
   wherein the Pd content is 1000 ppm or less.

### Advantageous Effects of Invention

The present disclosure provides, for example, a useful method for producing a fluoroalkyl aromatic compound, and a compound obtained by the production method.

### Description of Embodiments

The above summary of the present disclosure is not intended to describe each disclosed embodiment or every implementation of the present disclosure.

The following description of the present disclosure more particularly exemplifies illustrative embodiments. In several places throughout the present disclosure, guidance is provided through lists of examples, and these examples can be used in various combinations. In each instance, the described list serves only as a representative group, and should not be interpreted as an exclusive list.

All publications, patents, and patent applications cited herein are incorporated herein by reference in their entirety.

### 1. Terms

Unless otherwise specified, the symbols and abbreviations herein can be understood in the context of the present specification in the meanings commonly used in the art to which the present disclosure belongs.

The term "contains" as used herein is intended to include "consisting essentially of" and "consisting of."

Unless otherwise specified, the steps, treatments, or operations described herein can be performed at room temperature. The room temperature referred to herein can mean a temperature in the range of 10 to 40°C.

The notation "Cₙ₋ₘ" (where n and m are each an integer of 1 or more, and n<m) used herein means that the number of carbon atoms is n or more and m or less, as is usually understood by persons skilled in the art.

Examples of the "halogen" in the present specification include fluorine (F), chlorine (Cl), bromine (Br), and iodine (I).

Examples of the "alkyl group" in the present specification include linear or branched C₁₋₂₀ alkyl groups, such as methyl, ethyl, propyl (e.g., n-propyl and isopropyl), butyl (e.g., n-butyl, isobutyl, sec-butyl, and tert-butyl), pentyl, and hexyl.

Examples of the "alkoxy group" in the present specification include linear or branched C₁₋₂₀ alkoxy groups, such as methoxy, ethoxy, propoxy (e.g., n-propoxy and isopropoxy), butoxy (e.g., n-butoxy, isobutoxy, sec-butoxy, and tert-butoxy), pentyloxy, and hexyloxy.

Examples of the "alkylthio group" in the present specification include linear or branched C₁₋₂₀ alkylthio groups, such as methylthio, ethylthio, propylthio (e.g., n-propylthio and isopropylthio), butylthio (e.g., n-butylthio, isobutylthio, sec-butylthio, and tert-butylthio), pentylthio, and hexylthio.

Examples of the "cycloalkyl group" in the present specification include C₃₋₂₀ cycloalkyl groups, such as cyclopropyl, cyclobutyl, cyclopentyl, cyclohexyl, and cycloheptyl.

The "aryl group" in the present specification can be, for example, monocyclic, bicyclic, tricyclic, or tetracyclic. Examples of the "aryl group" include C₆₋₂₀ aryl groups, such as phenyl, 1-naphthyl, 2-naphthyl, 2-biphenyl, 3-biphenyl, 4-biphenyl, and 2-anthryl.

The "heteroaryl group" in the present specification can be monocyclic, bicyclic, tricyclic, or tetracyclic. The "heteroaryl group" can be, for example, a 5- to 18-membered heteroaryl group. The "heteroaryl group" can be, for example, a heteroaryl group containing, in addition to carbon atoms, one, two, three, or four heteroatoms selected from oxygen, sulfur, and nitrogen atoms as a ring-constituting atom. The "heteroaryl group" includes "monocyclic heteroaryl groups" and "aromatic fused heterocyclic groups."

Examples of the "monocyclic heteroaryl groups" include pyrrolyl (e.g., 1-pyrrolyl, 2-pyrrolyl, and 3-pyrrolyl), furyl (e.g., 2-furyl and 3-furyl), thienyl (e.g., 2-thienyl and 3-thienyl), pyrazolyl (e.g., 1-pyrazolyl, 3-pyrazolyl, and 4-pyrazolyl), imidazolyl (e.g., 1-imidazolyl, 2-imidazolyl, and 4-imidazolyl), isoxazolyl (e.g., 3-isoxazolyl, 4-isoxazolyl, and 5-isoxazolyl), oxazolyl (e.g., 2-oxazolyl, 4-oxazolyl, and 5-oxazolyl), isothiazolyl (e.g., 3-isothiazolyl, 4-isothiazolyl, and 5-isothiazolyl), thiazolyl (e.g., 2-thiazolyl, 4-thiazolyl, and 5-thiazolyl), triazolyl (e.g., 1,2,3-triazol-3-yl and 1,2,4-triazol-4-yl), oxadiazolyl (e.g., 1,2,4-oxadiazol-3-yl and 1,2,4-oxadiazol-5-yl), thiadiazolyl (e.g., 1,2,4-thiadiazol-3-yl and 1,2,4-thiadiazol-5-yl), tetrazolyl, pyridyl (e.g., 2-pyridyl, 3-pyridyl, and 4-pyridyl), pyridazinyl (e.g., 3-pyridazinyl and 4-pyridazinyl), pyrimidinyl (e.g., 2-pyrimidinyl, 4-pyrimidinyl, and 5-pyrimidinyl), pyrazinyl, and the like.

Examples of the "aromatic fused heterocyclic groups" include isoindolyl (e.g., 1-isoindolyl, 2-isoindolyl, 3-isoindolyl, 4-isoindolyl, 5-isoindolyl, 6-isoindolyl, and 7-isoindolyl), indolyl (e.g., 1-indolyl, 2-indolyl, 3-indolyl, 4-indolyl, 5-indolyl, 6-indolyl, and 7-indolyl), benzo[b]furanyl (e.g., 2-benzo[b]furanyl, 3-benzo[b]furanyl, 4-benzo[b]furanyl, 5-benzo[b]furanyl, 6-benzo[b]furanyl, and 7-benzo[b]furanyl), benzo[c]furanyl (e.g., 1-benzo[c]furanyl, 4-benzo[c]furanyl, and 5-benzo[c]furanyl), benzo[b]thienyl (e.g., 2-benzo[b]thienyl, 3-benzo[b]thienyl, 4-benzo[b]thienyl, 5-benzo[b]thienyl, 6-benzo[b]thienyl, and 7-benzo[b]thienyl), benzo[c]thienyl (e.g., 1-benzo[c]thienyl, 4-benzo[c]thienyl, and 5-benzo[c]thienyl), indazolyl (e.g., 1-indazolyl, 2-indazolyl, 3-indazolyl, 4-indazolyl, 5-indazolyl, 6-indazolyl, and 7-indazolyl), benzimidazolyl (e.g., 1-benzimidazolyl, 2-benzimidazolyl, 4-benzimidazolyl, and 5-benzimidazolyl), 1,2-benzisoxazolyl (e.g., 1,2-benzisoxazol-3-yl, 1,2-benzisoxazol-4-yl, 1,2-benzisoxazol-5-yl, 1,2-benzisoxazol-6-yl, and 1,2-benzisoxazol-7-yl), benzoxazolyl (e.g., 2-benzoxazolyl, 4-benzoxazolyl, 5-benzoxazolyl, 6-benzoxazolyl, and 7-benzoxazolyl), 1,2-benzisothiazolyl (e.g., 1,2-benzisothiazol-3-yl, 1,2-benzisothiazol-4-yl, 1,2-benzisothiazol-5-yl, 1,2-benzisothiazol-6-yl, and 1,2-benzisothiazol-7-yl), benzothiazolyl (e.g., 2-benzothiazolyl, 4-benzothiazolyl, 5-benzothiazolyl, 6-benzothiazolyl, and 7-benzothiazolyl), isoquinolyl (e.g., 1-isoquinolyl, 3-isoquinolyl, 4-isoquinolyl, and 5-isoquinolyl), quinolyl (e.g., 2-quinolyl, 3-quinolyl, 4-quinolyl, 5-quinolyl, and 8-quinolyl), cinnolinyl (e.g., 3-cinnolinyl, 4-cinnolinyl, 5-cinnolinyl, 6-cinnolinyl, 7-cinnolinyl, and 8-cinnolinyl), phthalazinyl (e.g., 1-phthalazinyl, 4-phthalazinyl, 5-phthalazinyl, 6-phthalazinyl, 7-phthalazinyl, and 8-phthalazinyl), quinazolinyl (e.g., 2-quinazolinyl, 4-quinazolinyl, 5-quinazolinyl, 6-quinazolinyl, 7-quinazolinyl, and 8-quinazolinyl), quinoxalinyl (e.g., 2-quinoxalinyl, 3-quinoxalinyl, 5-quinoxalinyl, 6-quinoxalinyl, 7-quinoxalinyl, and 8-quinoxalinyl), pyrazolo[1,5-a]pyridyl (e.g., pyrazolo[1,5-a]pyridin-2-yl, pyrazolo[1,5-a]pyridin-3-yl, pyrazolo[1,5-a]pyridin-4-yl, pyrazolo[1,5-a]pyridin-5-yl, pyrazolo[1,5-a]pyridin-6-yl, and pyrazolo[1,5-a]pyridin-7-yl), imidazo[1,2-a]pyridyl (e.g., imidazo[1,2-a]pyridin-2-yl, imidazo[1,2-a]pyridin-3-yl, imidazo[1,2-a]pyridin-5-yl, imidazo[1,2-a]pyridin-6-yl, imidazo[1,2-a]pyridin-7-yl, and imidazo[1,2-a]pyridin-8-yl), and the like.

In the "alkyl group optionally having one or more substituents" in the present specification, -CH₂-, which may be present in the alkyl group, is optionally replaced with -O-, -S-, or -NH-. Examples of the substituents for the alkyl group include halogen, a hydroxy group, an alkoxy group, a mercapto group, an alkylthio group, an alkoxycarbonyl (alkyl-O-CO-) group, an alkylcarbonyloxy (alkyl-CO-O-) group, an aryl group, and a combination of two or more thereof (e.g., a haloalkoxy group). When the alkyl group has a substituent, the number of substituents may be selected from the range of one to the maximum replaceable number, and can be, for example, 1, 2, 3, 4, or 5. When the number of substituents is two or more, the substituents may be the same or different.

Examples of the "alkyl group optionally having one or more substituents" include an alkyl group optionally substituted with one or more halogens (e.g., fluorine). An alkyl group substituted with one or more halogens is referred to as a "haloalkyl group." For example, when the halogen as a substituent is fluorine, the alkyl group may be referred to as a "fluoroalkyl group." The "fluoroalkyl group" may be a perfluoroalkyl group or a non-perfluoroalkyl group. Examples of the "fluoroalkyl group" include linear or branched fluoro-C₁₋₂₀ alkyl groups, such as a trifluoromethyl group, a perfluoroethyl group, a perfluoropropyl group (e.g., a perfluoro n-propyl group and a perfluoroisopropyl group), a perfluorobutyl group, a perfluoropentyl group, and a perfluorohexyl group. Examples of the "fluoroalkyl group optionally having one or more substituents" include, in addition to the fluoroalkyl groups mentioned above, fluoroalkyl groups substituted with one or more substituents (e.g., an alkoxy group; a haloalkoxy group, such as a fluoroalkoxy group; an alkoxycarbonyl group; and a haloalkoxycarbonyl group, such as a fluoroalkoxycarbonyl group). Specific examples thereof include linear or branched fluoro-C₁₋₂₀ alkyl groups substituted with one or more substituents, such as CF₃-O-CF₂-, CF₃-O-CF(CF₃)-, CF₃-O-CH₂-CH₂-, CF₃-O-CH(CF₃)-CH₂-, CF₃-O-CF₂-CF₂-, CF₃-CF₂-O-CF₂-, CF₃-CF₂-O-CF₂-CF₂-, CF₃-O-CF₂-O-CF₂-, CF₃-CF₂-CF₂-O-CH₂-CF₂-, CF₃-CF₂-CF₂-O-CF₂-CF₂-, CF₃-CF₂-CF₂-O-CF(CF₃)-CF₂-, CF₃-CF₂-CF₂-O-CF(CF₃)-CF₂-O-CF(CF₃)-CF₂-, and CF₃-CF₂-CF₂-O-[CF(CF₃)-CF₂-O-]₂-CF(CF₃)-CF₂-.

Examples of the substituents in the "aryl group optionally having one or more substituents" and the "heteroaryl group optionally having one or more substituents" in the present specification include halogen, a hydroxy group, an alkyl group, an alkoxy group, an alkylthio group, and a combination of two or more thereof (e.g., a haloalkyl group and a haloalkoxy group). When the aryl group has a substituent, the number of substituents may be selected from the range of one to the maximum replaceable number, and can be, for example, 1, 2, 3, 4, or 5. When the number of substituents is two or more, the substituents may be the same or different.

The "alkylene group," "cycloalkylene group," and "arylene group" in the present specification are divalent groups formed by removing one H from an "alkyl group," "cycloalkyl group," and "aryl group," respectively.

### 2. Method for Producing Compound Represented by Formula (2)

In one embodiment of the present disclosure, a method for producing a compound represented by formula (2) (which may be referred to as a "fluoroalkyl copper reagent") comprises step A of reacting a compound represented by formula (1), a copper compound, and a ligand in the presence of a reducing agent.

R^{F}-X¹ (1)

M-R^{F} (2)

In the formulas,
R^{F} is a fluoroalkyl group optionally having one or more substituents,
X¹ is Cl, Br, or I, and
M is Cu having a ligand.

### Compound Represented by Formula (1)

In R^{F}, the lower limit of the number of carbon atoms in the fluoroalkyl group may be, for example, 1, 2, or 3, and the upper limit may be, for example, 20, 16, 14, 12, or 10. R^{F} is preferably a fluoro-C₁₋₂₀ alkyl group optionally having one or more substituents (e.g., a perfluoro-C₁₋₂₀ alkyl group), more preferably a fluoro-C₁₋₁₂ alkyl group optionally having one or more substituents (e.g., a perfluoro-C₁₋₁₂ alkyl group), and even more preferably a fluoro-C₁₋₁₀ alkyl group optionally having one or more substituents (e.g., a perfluoro-C₁₋₁₀ alkyl group).

X¹ is preferably Br or I.

In one embodiment, X¹ is preferably Br.

In another embodiment, X¹ is preferably I.

### Copper Compound

The copper compound is not limited as long as it contains copper. The copper compound may be, for example, copper alone, or a monovalent or divalent copper compound. In one embodiment, the copper compound is preferably a monovalent copper compound.

Examples of the copper compound include copper powder; copper(I) halides, such as copper(I) iodide; copper(I) oxide; copper(I) sulfide; copper(I) hydroxide; copper(I) acetate; copper(II) halides, such as copper(II) iodide; copper(II) oxide; copper(II) sulfate; copper(II) hydroxide; copper(II) acetate; and the like. These copper compounds may be used in combination with an alkoxide, aryloxide, thioalkoxide, or thioaryloxide of an alkali metal, such as lithium, sodium, or potassium. Alternatively, the copper compound for use may be an alkoxide, aryloxide, thioalkoxide, or thioaryloxide of copper. Examples of the alkoxide or aryloxide include C₁₋₆ alkoxides, such as methoxide, ethoxide, propoxide, and butoxide (e.g., t-butoxide), and C₆₋₁₀ aryloxides, such as phenoxide. The thioalkoxide or thioaryloxide may be, for example, an alkoxide or aryloxide mentioned above in which O is replaced with S.

The amount of the copper compound for use is, for example, 0.5 mol or more, preferably 1 mol or more, and more preferably 1.5 mol or more, per mol of the compound represented by formula (1). The amount of the copper compound for use is, for example, 5 mol or less, preferably 4 mol or less, and more preferably 3 mol or less, per mol of the compound represented by formula (1). The amount of the copper compound for use is, for example, within the range of 0.5 to 5 mol, preferably within the range of 1 to 4 mol, and more preferably within the range of 1.5 to 3 mol, per mol of the compound represented by formula (1).

### Ligand

The ligand is not limited as long as it can be coordinated to copper. Examples of the ligand include pyridine-containing ligands, phosphine ligands, and the like.

Examples of the pyridine-containing ligands include phenanthroline (e.g., 1,10-phenanthroline), 2,2'-bipyridyl, pyridine, methylpyridine, lutidine (e.g., 2,6-lutidine), and the like.

The phosphine ligand is preferably a trialkylphosphine or a triarylphosphine. Examples of trialkylphosphines include tri(C₃₋₂₀ alkyl)phosphines, such as triisopropylphosphine, di-t-butylmethylphosphine, tri-t-butylphosphine, trihexylphosphine, tricyclopentylphosphine, tricyclohexylphosphine, triadamantylphosphine, tribicyclo[2,2,2]octylphosphine, and trinorbornylphosphine, and the like. Examples of triarylphosphines include tri(monocyclic aryl)phosphines, such as triphenylphosphine, trimesitylphosphine, and tri(o-tolyl)phosphine. Of these, triphenylphosphine, tricyclohexylphosphine, and tri-t-butylphosphine are preferred.

In one embodiment, the ligand is preferably a bidentate ligand. Preferable examples of bidentate ligands include 1,10-phenanthroline and the like.

The amount of the ligand for use is, for example, 0.5 mol or more, preferably 1 mol or more, and more preferably 1.5 mol or more, per mol of the compound represented by formula (1). The amount of the ligand for use is, for example, 5 mol or less, preferably 4 mol or less, and more preferably 3 mol or less, per mol of the compound represented by formula (1). The amount of the ligand for use is, for example, within the range of 0.5 to 5 mol, preferably within the range of 1 to 4 mol, and more preferably within the range of 1.5 to 3 mol, per mol of the compound represented by formula (1).

### Reducing Agent

By performing the reaction of step A in the presence of a reducing agent, the reaction can be performed at a low temperature, whereby the use of a high-boiling point solvent and associated complicated operations for removal can be avoided, making it possible to easily synthesize even a compound that is easily decomposed at high temperatures.

The reducing agent is not limited as long as it has reducing ability. In one embodiment, the reducing agent is preferably a compound containing boron and/or silicon. Examples of the reducing agent include diboron compounds, silylborane compounds, disilane compounds, and the like.

Examples of the diboron compound include a compound represented by formula (A): 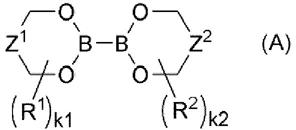 wherein
Z¹ and Z² are each independently a single bond, a double bond, or CH₂,
R¹ and R² are each independently an organic group,
two adjacent R¹s, when present, are optionally bonded together to form a ring,
two adjacent R²s, when present, are optionally bonded together to form a ring, and
k1 and k2 are each independently an integer of 0 or 1 or more.

In one embodiment, Z¹ and Z² are preferably both single bonds.

In another embodiment, Z¹ and Z² are preferably both double bonds.

In yet another embodiment, Z¹ and Z² are preferably both CH₂.

R¹ and R² are preferably each independently an alkyl group, more preferably a C₁₋₆ alkyl group, even more preferably a C₁₋₄ alkyl group, and still more preferably a C₁₋₂ alkyl group.

When Z¹ and Z² are single bonds, k1 and k2 are each 0, 1, 2, 3, or 4. When Z¹ and Z² are double bonds, k1 and k2 are each 0, 1, or 2. When Z¹ and Z² are CH₂, k1 and k2 are each 0, 1, 2, 3, 4, 5, or 6.

The compound represented by formula (A) may be, for example, a compound represented by formula (A1), (A2), (A3), or (A4):

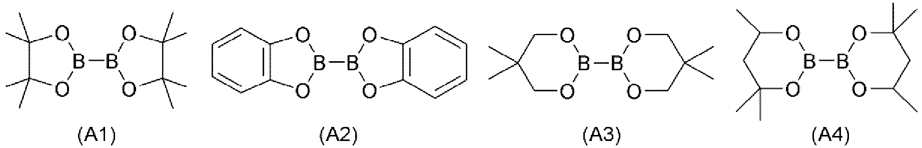

Examples of the silylborane compound include a compound represented by formula (B): 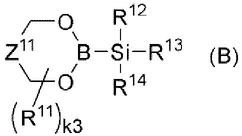 wherein
Z¹¹ is a single bond, a double bond, or CH₂,
R¹¹ is an organic group,
two adjacent R¹¹s, when present, are optionally bonded together to form a ring,
R¹², R¹³, and R¹⁴ are each independently an organic group, and
k3 is an integer of 0 or 1 or more.

In one embodiment, Z¹¹ is preferably a single bond.

In another embodiment, Z¹¹ is preferably a double bond.

In yet another embodiment, Z¹¹ is preferably CH₂.

R¹¹ is preferably a hydrocarbon group, more preferably an alkyl group, even more preferably a C₁₋₆ alkyl group, still more preferably a C₁₋₄ alkyl group, and particularly preferably a C₁₋₂ alkyl group.

R¹², R¹³, and R¹⁴ are preferably each independently an alkyl group or an aryl group, more preferably a C₁₋₆ alkyl group or a C₆₋₁₄ aryl group, even more preferably a C₁₋₄ alkyl group or a C₆₋₁₂ aryl group, and still more preferably a C₁₋₂ alkyl group or a C₆₋₁₀ aryl group.

When Z¹¹ is a single bond, k3 is 0, 1, 2, 3, or 4. When Z¹¹ is a double bond, k3 is 0, 1, or 2. When Z¹¹ is CH₂, k3 is 0, 1, 2, 3, 4, 5, or 6.

The compound represented by formula (B) may be, for example, a compound represented by formula (B1):

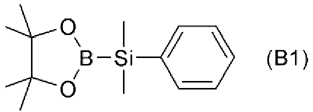

Examples of the disilane compound include a compound represented by formula (C): 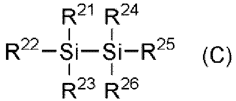 wherein
R²¹, R²², R²³, R²⁴, R²⁵, and R²⁶ are each independently H, halogen,
or an organic group.

R²¹, R²², R²³, R²⁴, R²⁵, and R²⁶ are preferably each independently H, halogen, an alkyl group, an alkoxy group, or an aryl group, more preferably H, halogen, a C₁₋₆ alkyl group, a C₁₋₆ alkoxy group, or a C₆₋₁₄ aryl group, even more preferably H, halogen, a C₁₋₄ alkyl group, a C₁₋₄ alkoxy group, or a C₆₋₁₂ aryl group, and still more preferably H, halogen (particularly Cl), a C₁₋₂ alkyl group, a C₁₋₂ alkoxy group, or a C₆₋₁₀ aryl group.

The compound represented by formula (C) may be, for example, a compound represented by formula (C1), (C2), (C3), (C4), (C5), (C6), (C7), (C8), or (C9):

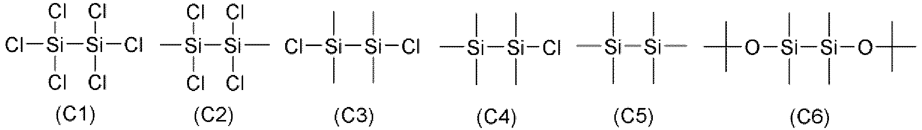

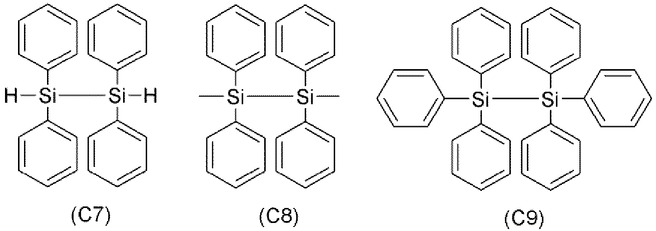

The amount of the reducing agent for use is, for example, 0.7 mol or more, preferably 0.8 mol or more, and more preferably 0.9 mol or more, per mol of the compound represented by formula (1). The amount of the reducing agent for use is, for example, 3 mol or less, preferably 2 mol or less, and more preferably 1.5 mol or less, per mol of the compound represented by formula (1). The amount of the reducing agent for use is, for example, within the range of 0.7 to 3 mol, preferably within the range of 0.8 to 1 mol, and more preferably within the range of 0.9 to 1.5 mol, per mol of the compound represented by formula (1).

### Organic Solvent

The reaction of step A is preferably performed in the presence of an organic solvent. In one embodiment, it is preferable that the organic solvent is not a high-boiling point solvent (or is a low-boiling point solvent) and has a boiling point under ordinary pressure of 150°C or lower, 140°C or lower, 130°C or lower, 120°C or lower, or 110°C or lower. Examples of the organic solvent include ether solvents (e.g., cyclic ether solvents, such as tetrahydrofuran), aromatic hydrocarbon solvents (e.g., toluene and xylene), mixed solvents of two or more of these, and the like.

The amount of the organic solvent for use is, for example, 0.5 mL or more, preferably 1 mL or more, and more preferably 1.5 mL or more, per mmol of the compound represented by formula (1). The amount of the organic solvent for use is, for example, 40 mL or less, preferably 30 mL or less, and more preferably 20 mL or less, per mmol of the compound represented by formula (1). The amount of the organic solvent for use is, for example, within the range of 0.5 to 40 mL, preferably within the range of 1 to 30 mL, and more preferably within the range of 1.5 to 20 mL, per mmol of the compound represented by formula (1).

### Reaction Temperature and Reaction Time

The reaction temperature and reaction time in step A are not limited as long as the reaction proceeds.

The reaction temperature is, for example, -30°C or higher, preferably -10°C or higher, and more preferably 0°C or higher. The reaction temperature is, for example, 40°C or lower, preferably 35°C or lower, and more preferably 30°C or lower. The reaction temperature is, for example, within the range of -30 to 40°C, preferably within the range of -10 to 35°C, and more preferably within the range of 0 to 30°C.

The reaction time is, for example, 5 minutes or more, preferably 10 minutes or more, more preferably 30 minutes or more, and even more preferably 1 hour or more. The reaction time is, for example, 48 hours or less, preferably 24 hours or less, and more preferably 12 hours or less. The reaction time is, for example, within the range of 5 minutes to 48 hours, preferably within the range of 10 minutes to 24 hours, and more preferably within the range of 1 to 12 hours.

### Optional Additional Step

The production method may further comprise isolating or purifying the compound represented by formula (2). The compound represented by formula (2) can be isolated or purified by a method, such as filtration, extraction, concentration, or chromatography, or a combination of these methods.

### 3. Method for Producing Compound Represented by Formula (4)

In one embodiment of the present disclosure, a method for producing a compound represented by formula (4) comprises step A and then step B of reacting a reaction product of step A (particularly the compound represented by formula (2)) and a compound represented by formula (3).

Ar-X² (3)

Ar-R^{F} (4)

In the formulas,
Ar is an aryl group optionally having one or more substituents or a heteroaryl group optionally having one or more substituents,
X² is Cl, Br, or I, and
R^{F} is as defined above.

### Compound Represented by Formula (3)

Ar is preferably a C₆₋₁₄ aryl group optionally having one or more substituents, or a 5- to 14-membered heteroaryl group optionally having one or more substituents, more preferably a C₆₋₁₂ aryl group optionally having one or more substituents, or a 5- to 12-membered heteroaryl group optionally having one or more substituents, and even more preferably a C₆₋₁₀ aryl group optionally having one or more substituents, or a 5- to 10-membered heteroaryl group optionally having one or more substituents. The heteroatom(s) contained in the heteroaryl groups are preferably selected from the group consisting of O, S, and N.

In one embodiment, Ar is preferably an aryl group optionally having one or more substituents. The substituent may be an organic group. Specific examples include an alkyl group optionally having one or more further substituents, an aryl group optionally having one or more further substituents, a heteroaryl group optionally having one or more further substituents, a carbonyl group (-L¹-CO-Q¹ or -CO-L¹-Q¹; Q¹ is H or a hydrocarbon group optionally having one or more further substituents, and L¹ is a single bond or -O-), an amide group (-L²-CO-NQ²Q³ or -NQ⁴-CO-L³-Q⁵; Q², Q³, Q⁴, and Q⁵ are each independently H or a hydrocarbon group optionally having one or more further substituents, and L² and L³ are each independently a single bond or -O-), an alkoxy group, a hydroxy group, halogen (a halogeno group), and combinations thereof. In one embodiment, the substituent is preferably fluorine and/or a fluoroalkyl group, and the substituent may be a fluoroalkyl group.

X² is preferably Br or I.

In one embodiment, X² is preferably Br.

In another embodiment, X² is preferably I.

In one embodiment, the compound represented by formula (3) is preferably a compound represented by formula (3A) or formula (3B):

X²¹-Ar¹-A⁰-Ar²-X²² (3A)

or

X²³-Ar³-X²⁴ (3B),

wherein
Ar¹, Ar², and Ar³ are each independently an arylene group optionally having one or more substituents, or a heteroarylene group optionally having one or more substituents,
A⁰ is a single bond, -O-, -S-, an alkylene group optionally having one or more substituents, a cycloalkylene group optionally having one or more substituents, or an arylene group optionally having one or more substituents, and
X²¹, X²², X²³, and X²⁴ are each independently Cl, Br, or I.

Ar¹ and Ar² may be the same or different. Preferable examples of Ar¹, Ar², and Ar³ include divalent groups corresponding to the preferable examples of Ar.

When A⁰ is an alkylene group optionally having one or more substituents, the lower limit of the number of carbon atoms in the alkylene group can be, for example, 1 or 2, and the upper limit can be, for example, 6, 5, 4, 3, or 2. A⁰ is preferably a C₁₋₆ alkylene group optionally having one or more substituents, more preferably a C₁₋₆ alkylene group optionally having one or more substituents, even more preferably a C₁₋₄ alkylene group optionally having one or more substituents, and still more preferably a C₁₋₂ alkylene group optionally having one or more substituents. The substituent may be any organic group, and specific examples include an alkyl group optionally having one or more further substituents, an aryl group optionally having one or more further substituents, a heteroaryl group optionally having one or more further substituents, a carbonyl group (-L¹-CO-Q¹ or - CO-L¹-Q¹), an amide group (-L²-CO-NQ²Q³ or -NQ⁴-CO-L³-Q⁵), an alkoxy group, a hydroxy group, halogen (a halogeno group), and combinations thereof.

When A⁰ is a cycloalkylene group optionally having one or more substituents, A⁰ is preferably a C₅₋₁₄ cycloalkylene group optionally having one or more substituents, more preferably a C₅₋₁₂ cycloalkylene group optionally having one or more substituents, and even more preferably a C₅₋₁₀ cycloalkylene group optionally having one or more substituents.

When A⁰ is an arylene group optionally having one or more substituents, A⁰ is preferably a C₆₋₁₄ arylene group optionally having one or more substituents, more preferably a C₆₋₁₂ arylene group optionally having one or more substituents, and even more preferably a C₅₋₁₀ arylene group optionally having one or more substituents.

It is preferable that X²¹, X²², X²³, and X²⁴ are each independently Br or I. X²¹ and X²² may be the same or different. X²³ and X²⁴ may be the same or different.

The amount of the compound represented by formula (3) for use (particularly the total amount of Cl, Br, and I) is, for example, 0.05 mol or more, preferably 0.1 mol or more, and more preferably 0.15 mol or more, per mol of the compound represented by formula (2). The amount of the compound represented by formula (3) for use is, for example, 3 mol or less, preferably 2 mol or less, and more preferably 1.5 mol or less, per mol of the compound represented by formula (2). The amount of the compound represented by formula (3) for use is, for example, within the range of 0.05 to 3 mol, preferably within the range of 0.1 to 2 mol, and more preferably within the range of 0.15 to 1.5 mol, per mol of the compound represented by formula (2).

### Organic Solvent

The reaction of step B is preferably performed in the presence of an organic solvent. In one embodiment, it is preferable that the organic solvent is not a high-boiling point solvent (or is a low-boiling point solvent) and has a boiling point under ordinary pressure of 150°C or lower, 140°C or lower, 130°C or lower, 120°C or lower, or 110°C or lower. Examples of the organic solvent include ether solvents (e.g., cyclic ether solvents, such as tetrahydrofuran), aromatic hydrocarbon solvents (e.g., toluene and xylene), mixed solvents of two or more of these, and the like. The organic solvent may be different from the organic solvent that can be used in step A, but is preferably the same.

### Reaction Temperature and Reaction Time

The reaction temperature and reaction time in step B are not limited as long as the reaction proceeds.

The reaction temperature is, for example, 0°C or higher, preferably 10°C or higher, and more preferably 20°C or higher. The reaction temperature is, for example, 125°C or lower, preferably 100°C or lower, and more preferably 80°C or lower. The reaction temperature is, for example, within the range of 0 to 125°C, preferably within the range of 10 to 100°C, and more preferably within the range of 20 to 80°C.

The reaction time is, for example, 30 minutes or more, preferably 1 hour or more, and more preferably 2 hours or more. The reaction time is, for example, 48 hours or less, preferably 24 hours or less, and more preferably 12 hours or less. The reaction time is, for example, within the range of 30 minutes to 48 hours, preferably within the range of 1 to 24 hours, and more preferably within the range of 2 to 12 hours.

In another embodiment of the present disclosure, a method for producing a compound represented by formula (4) comprises step C of reacting a compound represented by formula (1) and a compound represented by formula (3) in the presence of a reducing agent, a copper compound, and a ligand.

The compound represented by formula (1) and the compound represented by formula (3) may be the same compounds as those listed for step A and step B as examples, respectively.

The amount of the compound represented by formula (3) for use (particularly the total amount of Cl, Br, and I) is, for example, 0.05 mol or more, preferably 0.1 mol or more, and more preferably 0.15 mol or more, per mol of the compound represented by formula (1). The amount of the compound represented by formula (3) for use is, for example, 3 mol or less, preferably 2 mol or less, and more preferably 1.5 mol or less, per mol of the compound represented by formula (1). The amount of the compound represented by formula (3) for use is, for example, within the range of 0.05 to 3 mol, preferably within the range of 0.1 to 2 mol, and more preferably within the range of 0.15 to 1.5 mol, per mol of the compound represented by formula (1).

Examples of the reducing agent, copper compound, and ligand include the same as those listed for step A as examples. The amounts of the reducing agent, copper compound, and ligand for use relative to the compound represented by formula (1) can also be selected from the same ranges as those listed for step A as examples.

### Organic Solvent

The reaction of step C is preferably performed in the presence of an organic solvent. Examples of the organic solvent include the same organic solvents as those listed for step A as examples. The amount of the organic solvent for use relative to the compound represented by formula (1) can also be selected from the same ranges as those listed for step A as examples.

### Reaction Temperature and Reaction Time

The reaction temperature and reaction time in step C are not limited as long as the reaction proceeds. The reaction temperature and reaction time can be selected from the same ranges as those listed for step A as examples.

### Optional Additional step

In this production method, for example, when a compound represented by formula (3A) is used, a compound represented by formula (4B) and/or a compound represented by formula (4C) may be produced as by-products.

X²¹-Ar¹-A⁰-Ar²-R^{F} (4B)

R^{F}-Ar¹-A⁰-Ar²-X²² (4C)

The production method may further comprise isolating or purifying the compound represented by formula (4). The compound represented by formula (4) can be isolated or purified by a method, such as filtration, extraction, concentration, or chromatography, or a combination of these methods.

### 4. Compound Represented by Formula (4A)

A compound according to one embodiment of the present disclosure is a compound represented by formula (4A): 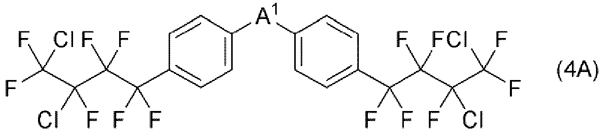 wherein
A¹ is a single bond, -O-, -S-, an alkylene group optionally having one or more substituents, a cycloalkylene group optionally having one or more substituents, or an arylene group optionally having one or more substituents.

When A¹ is an alkylene group optionally having one or more substituents, the lower limit of the number of carbon atoms in the alkylene group can be, for example, 1 or 2, and the upper limit can be, for example, 6, 5, 4, 3, or 2. A¹ is preferably a C₁₋₆ alkylene group optionally having one or more substituents, more preferably a C₁₋₆ alkylene group optionally having one or more substituents, even more preferably a C₁₋₄ alkylene group optionally having one or more substituents, and still more preferably a C₁₋₂ alkylene group optionally having one or more substituents. The substituent may be any organic group, and specific examples include an alkyl group optionally having one or more further substituents, an aryl group optionally having one or more further substituents, a heteroaryl group optionally having one or more further substituents, a carbonyl group (-L¹-CO-Q¹ or - CO-L¹-Q¹), an amide group (-L²-CO-NQ²Q³ or -NQ⁴-CO-L³-Q⁵), an alkoxy group, a hydroxy group, halogen (a halogeno group), and combinations thereof.

When A¹ is a cycloalkylene group optionally having one or more substituents, A¹ is preferably a C₅₋₁₄ cycloalkylene group optionally having one or more substituents, more preferably a C₅₋₁₂ cycloalkylene group optionally having one or more substituents, and even more preferably a C₅₋₁₀ cycloalkylene group optionally having one or more substituents.

When A¹ is an arylene group optionally having one or more substituents, A¹ is preferably a C₆₋₁₄ arylene group optionally having one or more substituents, more preferably a C₆₋₁₂ arylene group optionally having one or more substituents, and even more preferably a C₅₋₁₀ arylene group optionally having one or more substituents.

The compound represented by formula (4A) can be produced, for example, according to the production method for the compound represented by formula (4), by reacting a compound of formula (3A) in which Ar¹ and Ar² are phenylene groups, a compound of formula (1) in which R^{F} is CF₂Cl-CFCl-CF₂-CF₂-, a copper compound, and a ligand (or a compound of formula (2) in which R^{F} is CF₂Cl-CFCl-CF₂-CF₂- produced by reacting these).

### 5. Compound Represented by Formula (5A)

A compound according to one embodiment of the present disclosure is a compound represented by formula (5A): 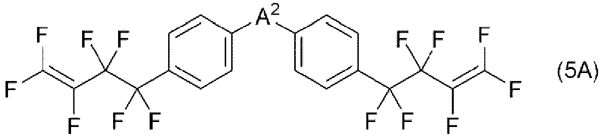 wherein
A² is a single bond, -O-, -S-, an alkylene group optionally having one or more substituents, a cycloalkylene group optionally having one or more substituents, or an arylene group optionally having one or more substituents.

When A² is an alkylene group optionally having one or more substituents, the lower limit of the number of carbon atoms in the alkylene group can be, for example, 1 or 2, and the upper limit can be, for example, 6, 5, 4, 3, or 2. A² is preferably a C₁₋₆ alkylene group optionally having one or more substituents, more preferably a C₁₋₆ alkylene group optionally having one or more substituents, even more preferably a C₁₋₄ alkylene group optionally having one or more substituents, and still more preferably a C₁₋₂ alkylene group optionally having one or more substituents. The substituent may be any organic group, and specific examples include an alkyl group optionally having one or more further substituents, an aryl group optionally having one or more further substituents, a heteroaryl group optionally having one or more further substituents, a carbonyl group (-L¹-CO-Q¹ or - CO-L¹-Q¹), an amide group (-L²-CO-NQ²Q³ or -NQ⁴-CO-L³-Q⁵), an alkoxy group, a hydroxy group, halogen (a halogeno group), and combinations thereof.

When A² is a cycloalkylene group optionally having one or more substituents, A² is preferably a C₅₋₁₄ cycloalkylene group optionally having one or more substituents, more preferably a C₅₋₁₂ cycloalkylene group optionally having one or more substituents, and even more preferably a C₅₋₁₀ cycloalkylene group optionally having one or more substituents.

When A² is an arylene group optionally having one or more substituents, A² is preferably a C₆₋₁₄ arylene group optionally having one or more substituents, more preferably a C₆₋₁₂ arylene group optionally having one or more substituents, and even more preferably a C₅₋₁₀ arylene group optionally having one or more substituents.

The compound represented by formula (5A) can be produced, for example, by subjecting the compound represented by formula (4A) to a dechlorination reaction. The dechlorination reaction can be performed under conditions known or conventional in the art.

The compound represented by formula (5A) may comprise Al, Cr, Cu, Fe, Ni, and Zn, or may not comprise a part or all of these metals. The content of each of Al, Cr, Cu, Fe, Ni, and Zn in the compound represented by formula (5A) is, for example, 1000 ppm or less, preferably 800 ppm or less, and more preferably 500 ppm or less. Since the lower the content of each of the above metals, the more preferable it is, the lower limit is not limited. The lower limit may be, for example, 0.001 ppm or more. The content of each of Al, Cr, Cu, Fe, Ni, and Zn can be measured by, for example, ICP (high-frequency inductively coupled plasma) emission analysis. In one embodiment, the content of each of Al, Cr, Cu, Fe, Ni, and Zn is preferably below the limit of detection by ICP emission analysis.

The total content of Al, Cr, Cu, Fe, Ni, and Zn in the compound represented by formula (5A) is, for example, 6000 ppm or less, preferably 4800 ppm or less, and more preferably 3000 ppm or less. In one embodiment, the total content of the above metals may be 1000 ppm or less, 800 ppm or less, or 500 ppm or less. Since the lower the total content of the above metals, the more preferable it is, the lower limit is not limited. The lower limit may be, for example, 0.001 ppm or more. The total content of Al, Cr, Cu, Fe, Ni, and Zn can be measured by, for example, ICP (high-frequency inductively coupled plasma) emission analysis. In one embodiment, the total content of Al, Cr, Cu, Fe, Ni, and Zn is preferably below the limit of detection by ICP emission analysis.

The compound represented by formula (5A) may comprise Pd or may not comprise Pd. The Pd content in the compound represented by formula (5A) is, for example, 1000 ppm or less, preferably 800 ppm or less, and more preferably 500 ppm or less. Since the lower the Pd content, the more preferable it is, the lower limit is not limited. The lower limit may be, for example, 0.001 ppm or more. The Pd content can be measured, for example, by ICP emission analysis. In one embodiment, the Pd content is preferably below the limit of detection by ICP emission analysis.

### 7. Compound Represented by Formula (5)

A compound according to one embodiment of the present disclosure is a compound represented by formula (5): 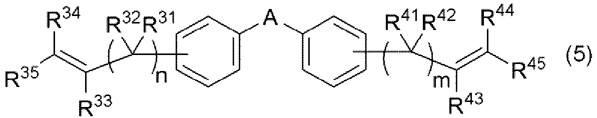 wherein
A is a single bond, -O-, -S-, an alkylene group optionally having one or more substituents, a cycloalkylene group optionally having one or more substituents, or an arylene group optionally having one or more substituents,
R³¹, R³², R³³, R³⁴, R³⁵, R⁴¹, R⁴², R⁴³, R⁴⁴, and R⁴⁵ are each independently fluorine, an alkyl group, or a fluoroalkyl group, and
n and m are each independently 0, 1, or 2.

When A is an alkylene group optionally having one or more substituents, the lower limit of the number of carbon atoms in the alkylene group can be, for example, 1 or 2, and the upper limit can be, for example, 6, 5, 4, 3, or 2. A is preferably a C₁₋₆ alkylene group optionally having one or more substituents, more preferably a C₁₋₆ alkylene group optionally having one or more substituents, even more preferably a C₁₋₄ alkylene group optionally having one or more substituents, and still more preferably a C₁₋₂ alkylene group optionally having one or more substituents. The substituent may be any organic group, and specific examples include an alkyl group optionally having one or more further substituents, an aryl group optionally having one or more further substituents, a heteroaryl group optionally having one or more further substituents, a carbonyl group (-L¹-CO-Q¹ or - CO-L¹-Q¹), an amide group (-L²-CO-NQ²Q³ or -NQ⁴-CO-L³-Q⁵), an alkoxy group, a hydroxy group, halogen (a halogeno group), and combinations thereof.

When A is a cycloalkylene group optionally having one or more substituents, A is preferably a C₅₋₁₄ cycloalkylene group optionally having one or more substituents, more preferably a C₅₋₁₂ cycloalkylene group optionally having one or more substituents, and even more preferably a C₅₋₁₀ cycloalkylene group optionally having one or more substituents.

When A is an arylene group optionally having one or more substituents, A is preferably a C₆₋₁₄ arylene group optionally having one or more substituents, more preferably a C₆₋₁₂ arylene group optionally having one or more substituents, and even more preferably a C₅₋₁₀ arylene group optionally having one or more substituents.

R³¹, R³², R³³, R³⁴, R³⁵, R⁴¹, R⁴², R⁴³, R⁴⁴, and R⁴⁵ are preferably each independently fluorine, a C₁₋₆ alkyl group, or a fluoro-C₁₋₆ alkyl group, more preferably fluorine, a C₁₋₄ alkyl group, or a fluoro-C₁₋₄ alkyl group, and even more preferably fluorine, a C₁₋₂ alkyl group, or a fluoro-C₁₋₂ alkyl group. In one embodiment, R³¹, R³², R³³, R³⁴, and R³⁵ are preferably the same as R⁴¹, R⁴², R⁴³, R⁴⁴, and R⁴⁵, respectively.

In one embodiment, n and m are preferably both 0.

In another embodiment, n and m are preferably both 1.

In yet another embodiment, n and m are preferably both 2.

The compound represented by formula (5) may comprise Al, Cr, Cu, Fe, Ni, and Zn, or may not comprise a part or all of these metals. The content of each of Al, Cr, Cu, Fe, Ni, and Zn in the compound represented by formula (5) is, for example, 1000 ppm or less, preferably 800 ppm or less, and more preferably 500 ppm or less. Since the lower the content of each of the above metals, the more preferable it is, the lower limit is not limited. The lower limit may be, for example, 0.001 ppm or more. The content of each of Al, Cr, Cu, Fe, Ni, and Zn can be measured by, for example, ICP (high-frequency inductively coupled plasma) emission analysis. In one embodiment, the content of each of Al, Cr, Cu, Fe, Ni, and Zn is preferably below the limit of detection by ICP emission analysis.

The total content of Al, Cr, Cu, Fe, Ni, and Zn in the compound represented by formula (5) is, for example, 6000 ppm or less, preferably 4800 ppm or less, and more preferably 3000 ppm or less. In one embodiment, the total content of the above metals may be 1000 ppm or less, 800 ppm or less, or 500 ppm or less. Since the lower the total content of the above metals, the more preferable it is, the lower limit is not limited. The lower limit may be, for example, 0.001 ppm or more. The total content of Al, Cr, Cu, Fe, Ni, and Zn can be measured by, for example, ICP (high-frequency inductively coupled plasma) emission analysis. In one embodiment, the total content of Al, Cr, Cu, Fe, Ni, and Zn is preferably below the limit of detection by ICP emission analysis.

The compound represented by formula (5) may comprise Pd or may not comprise Pd. The Pd content in the compound represented by formula (5) is, for example, 1000 ppm or less, preferably 800 ppm or less, and more preferably 500 ppm or less. Since the lower the Pd content, the more preferable it is, the lower limit is not limited. The lower limit may be, for example, 0.001 ppm or more. The Pd content can be measured, for example, by ICP emission analysis. In one embodiment, the Pd content is preferably below the limit of detection by ICP emission analysis.

The compound represented by formula (5) can be produced, for example, according to the production method for the compound represented by formula (4), by reacting a compound of formula (3A) in which Ar¹ and Ar² are phenylene groups, a compound of formula (1) in which R^{F} is CR³⁵R³⁴Cl-CR³³Cl-(CR³²R³¹)ₙ- and/or a compound of formula (1), in which R^{F} is CR⁴⁵R⁴⁴Cl-CR⁴³Cl-(CR⁴²R⁴¹)ₘ-, a copper compound, and a ligand (or a compound of formula (2) produced by reacting these), and subjecting the compound of formula (4) produced by the reaction above to a dechlorination reaction. The dechlorination reaction can be performed under conditions known or conventional in the art.

### Examples

One embodiment of the present disclosure is described in more detail below by way of the Examples; however, the present disclosure is not limited thereto. The abbreviations used in the Examples are as follows.
B₂pin₂: Bis(pinacolato)diboron
DMF: N,N-dimethylformamide
HPLC: High-performance liquid chromatography
PhMe₂SiBpin: (Dimethylphenylsilyl)boronic acid pinacol ester
tBu: t-Butyl
THF: Tetrahydrofuran

### Example 1

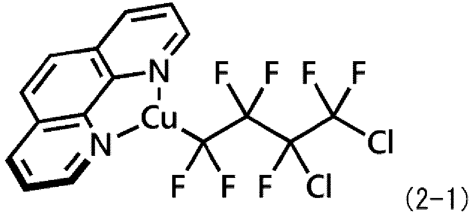

In a nitrogen atmosphere, a solution of CuOtBu (1 mmol, 134 mg) and 1,10-phenanthrorine (1 mmol, 180 mg) in THF (4 mL) was prepared in a screw-cap test tube. A THF solution (1 mL) of B₂pin₂ (0.5 mmol, 127 mg) and ICF₂CF₂CFClCF₂Cl (0.5 mmol, 189 mg) was added dropwise. The reaction solution was stirred at room temperature for 10 minutes and then filtered. The filtrate was concentrated and washed with ether to give 197.6 mg of the title compound as a brown solid in a yield of 80%.
¹H NMR (400 MHz, THF-d₈): δ9.1 (br, 2H), 8.7 (br, 2H), 8.1 (br, 2H), 8.0 (br, 2H).
¹⁹F NMR (376 MHz, THF-d₈): δ-65.7 (br, 2F), -111.5 (br, 2F), - 120.3 (br, 2F), -132.3 (br, 1F).

### Example 2

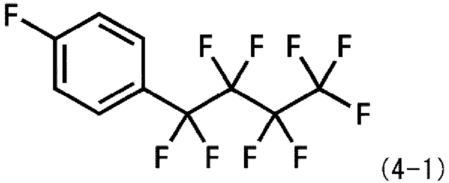

In a nitrogen atmosphere, a solution of CuOtBu (0.24 mmol, 32.1 mg) and 1,10-phenanthrorine (0.24 mmol, 43.2 mg) in THF (0.3 mL) was prepared in a screw-cap test tube. A THF solution (0.2 mL) of B₂pin₂ (0.12 mmol, 30.5 mg) and ICF₂CF₂CF₂CF₃ (0.12 mmol, 20 µL) was added dropwise. After the reaction solution was stirred at room temperature for 1 hour, 4-fluoroiodobenzene (0.1 mmol, 11.6 µL) was added, sealing was performed, and stirring was performed at 40°C for 18 hours to give the title compound in a yield of 82%.
¹⁹F NMR (376 MHz, CDCl₃): -107.1 (s, 1F), -109.9 (t, 13 Hz, 2F), - 122.5 (t, 9 Hz, 2F), -125.4 (m, 2F).

### Example 3

The same procedure was performed as described above except that B₂pin₂ was changed to PhMe₂SiBpin (0.12 mmol). The results confirmed the production of the title compound (4-1) in a yield of 63% by ¹⁹F NMR.

### Example 4

The same procedure was performed as described above except that THF was changed to diethyl ether. The results confirmed the production of compound (4-1) in a yield of 8% by ¹⁹F NMR.

### Example 5

The same procedure was performed as described above except that THF was changed to toluene. The results confirmed the production of compound (4-1) in a yield of 30% by ¹⁹F NMR.

### Example 6

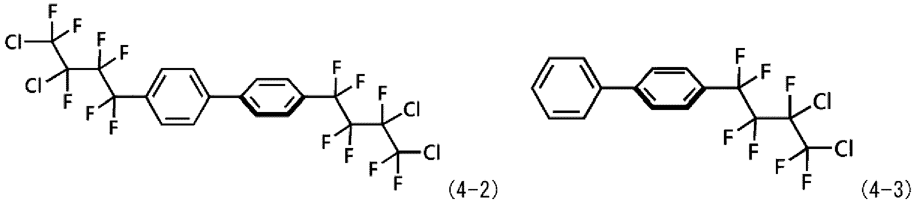

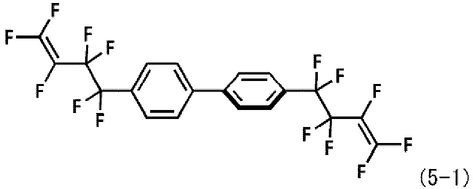

In a glove box, solution 1 of CuOtBu (4.8 mmol, 654.6 mg) and 1,10-phenanthrorine (4.8 mmol, 865.1 mg) in THF (4.8 mL) was prepared in a screw-cap test tube, and the solution was cooled to -30°C. THF solution 2 (2.4 mL) of B₂pin₂ (2.4 mmol, 610.1 mg) and ICF₂CF₂CFClCF₂Cl (2.4 mmol, 426 µL) was prepared. Solution 2 was slowly added dropwise to solution 1. The container for solution 2 was washed twice with THF (1.2 mL), and the washed liquid was added to solution 1. After the solution was stirred at room temperature for 1 hour, 4,4'-diiodobiphenyl (0.8 mmol, 325.3 mg) was added, sealing was performed, and stirring was performed at 40°C for 18 hours. After completion of the reaction, a mixture of compound (4-2) and compound (4-3) in a ratio of 10:1 was obtained. This mixture contained a small amount of compound (5-1). The mixture was filtered and purified by column chromatography to give compound (4-2) in a yield of 82%.
¹H NMR (400 MHz, CDCl₃): δ7.65 (m, 8H)
¹⁹F NMR (376 MHz, CDCl₃): δ-63.3 (t, 2F), δ-107.0, -107.7, -108.5, -109.2 (m, 2F), δ-112.6, -113.4, -114.0, -114.7 (m, 2F), δ-130.0 (m, 1F)GCMS m/z: 656

### Example 7

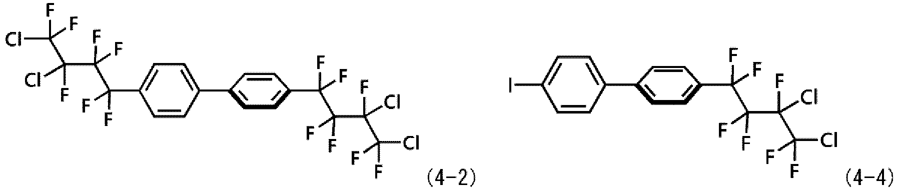

In a glove box, solution 1 of CuOtBu (4.8 mmol, 654.6 mg) and 1,10-phenanthrorine (4.8 mmol, 865.1 mg) in THF (4.8 mL) was prepared in a screw-cap test tube, and the solution was cooled to -30°C. THF solution 2 (2.4 mL) of B₂pin₂ (2.4 mmol, 610.1 mg) and ICF₂CF₂CFClCF₂Cl (2.4 mmol, 426 µL) was prepared. Solution 2 was slowly added dropwise to solution 1. The container for solution 2 was washed twice with THF (1.2 mL), and the washed liquid was added to solution 1. After the solution was stirred at room temperature for 1 hour, 4,4'-diiodobiphenyl (0.8 mmol, 325.3 mg), Zn (325 mg), ZnCl₂ (44 mg), and DMF (8.4 mL) were added, sealing was performed, and stirring was performed at 40°C for 18 hours. After completion of the reaction, 232 mg of a crude product was obtained. Analysis by NMR revealed that 4,4'-diiodobiphenyl, compound (4-2), and compound (4-4) were produced in a molar ratio of 0.7:1:1.4.

### Example 8

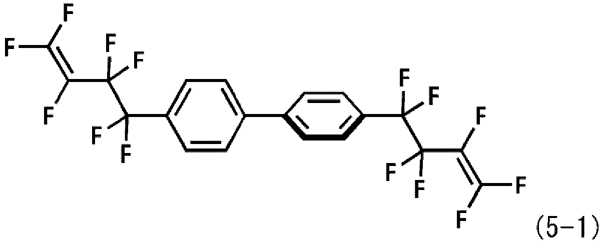

In a glove box, zinc powder (6.5 mmol, 438 mg) and zinc chloride (0.65 mmol, 87.9 mg) were weighed into a 50 mL two-necked round-bottom flask, and DMF (3 mL) was added. Compound (4-2) (0.65 mmol, 428.3 mg) was dissolved in DMF (3 mL), and the solution was added to the reaction solution. Washing was performed twice with DMF (2 mL), and the washed liquid was added to the reaction solution, followed by stirring at 60°C for 2 hours in a nitrogen atmosphere. After completion of the reaction, filtration and extraction, as well as purification by HPLC, were performed to give compound (5-1) in a yield of 93%.
¹H NMR (400 MHz, CDCl₃): δ7.63 (dd, 8H)¹⁹F NMR (MHz, CDCl₃) δ-89.9 (m, 1F), δ-106.6 (m, 1F), δ-112.5 (d, 2F), δ-118.2 (t, 2F), δ-187.1 (m, 1F)
GCMS m/z: 513

The results of analysis of the resulting compound (5-1) by ICP-AES (Pd detection limit: 1 ppm) revealed that no Pd was detected.

## Claims

1. A method for producing a compound represented by formula (2):
M-R^{F} (2),
wherein M is Cu having a ligand, and
R^{F} is a fluoroalkyl group optionally having one or more substituents;
the method comprising:
step A of reacting, in the presence of a reducing agent, a compound represented by formula (1):
R^{F}-X¹ (1),
wherein R^{F} is as defined above, and
X¹ is Cl, Br, or I;
a copper compound, and a ligand.

2. A method for producing a compound represented by formula (4):
Ar-R^{F} (4),
wherein Ar is an aryl group optionally having one or more substituents or a heteroaryl group optionally having one or more substituents, and
R^{F} is a fluoroalkyl group optionally having one or more substituents;
the method comprising:
step A of reacting, in the presence of a reducing agent, a compound represented by formula (1):
R^{F}-X¹ (1),
wherein R^{F} is as defined above, and
X¹ is Cl, Br, or I;
a copper compound, and a ligand; and
step B of reacting a reaction product of step A and a compound represented by formula (3):
Ar-X² (3),
wherein Ar is as defined above, and
X² is Cl, Br, or I.

3. A method for producing a compound represented by formula (4):
Ar-R^{F} (4),
wherein Ar is an aryl group optionally having one or more substituents or a heteroaryl group optionally having one or more substituents, and
R^{F} is a fluoroalkyl group optionally having one or more substituents;
the method comprising:
step C of reacting, in the presence of a reducing agent, a copper compound, and a ligand, a compound represented by formula (1):
R^{F}-X¹ (1),
wherein R^{F} is as defined above, and
X¹ is Cl, Br, or I;
and a compound represented by formula (3):
Ar-X² (3),
wherein Ar is as defined above, and
X² is Cl, Br, or I.

4. The production method according to any one of claims 1 to 3, wherein the reducing agent is a compound containing boron and/or silicon.

5. The production method according to any one of claims 1 to 4, wherein the reducing agent is at least one member selected from the group consisting of a diboron compound, a silylborane compound, and a disilane compound.

6. The production method according to claim 5,
wherein the diboron compound is a compound represented by formula (A): 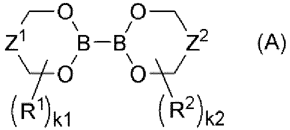
wherein Z¹ and Z² are each independently a single bond, a double bond, or CH₂,
R¹ and R² are each independently an alkyl group,
two adjacent R¹s, when present, are optionally bonded together to form a ring,
two adjacent R²s, when present, are optionally bonded together to form a ring, and
k1 and k2 are each independently an integer of 0 or 1 or more;
the silylborane compound is a compound represented by formula (B): 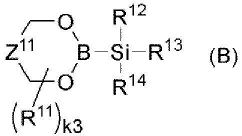
wherein Z¹¹ is a single bond, a double bond, or CH₂,
R¹¹ is an alkyl group,
two adjacent R¹¹s, when present, are optionally bonded together to form a ring,
R¹², R¹³, and R¹⁴ are each independently an alkyl group or an aryl group, and
k3 is an integer of 0 or 1 or more; and
the disilane compound is a compound represented by formula (C): 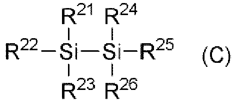 wherein R²¹, R²², R²³, R²⁴, R²⁵, and R²⁶ are each independently H, halogen, an alkyl group, an alkoxy group, or an aryl group.

7. The production method according to any one of claims 1 to 6, which is performed in an organic solvent.

8. The production method according to claim 7, wherein the organic solvent has a boiling point of 150°C or lower under ordinary pressure.

9. The production method according to claim 7 or 8, wherein the organic solvent is at least one member selected from the group consisting of ether solvents and aromatic hydrocarbon solvents.

10. The production method according to any one of claims 1 to 9, wherein the copper compound is a monovalent copper compound.

11. The production method according to any one of claims 1 to 10, wherein the copper compound is an alkoxide, aryloxide, thioalkoxide, or thioaryloxide of Cu.

12. The production method according to any one of claims 1 to 11, wherein the ligand is at least one member selected from the group consisting of pyridine-containing ligands and phosphine ligands.

13. The production method according to any one of claims 2 to 12, wherein step B or step C is performed at 50°C or lower.

14. The production method according to any one of claims 2 to 13, wherein Ar is an aryl group optionally having one or more substituents.

15. The production method according to any one of claims 2 to 14, wherein Ar is an aryl group having one or more fluorine atoms or fluoroalkyl groups.

16. A compound represented by formula (4A): 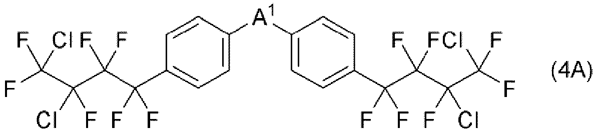 wherein A¹ is a single bond, -O-, -S-, an alkylene group optionally having one or more substituents, a cycloalkylene group optionally having one or more substituents, or an arylene group optionally having one or more substituents.

17. A compound represented by formula (5A): 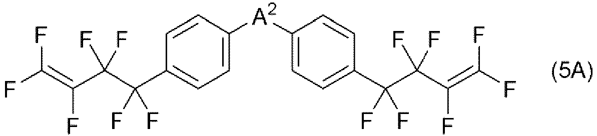 wherein A² is a single bond, -O-, -S-, an alkylene group optionally having one or more substituents, a cycloalkylene group optionally having one or more substituents, or an arylene group optionally having one or more substituents.

18. A compound represented by formula (5A): 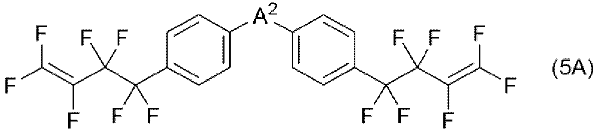
wherein A² is a single bond, -O-, -S-, an alkylene group optionally having one or more substituents, a cycloalkylene group optionally having one or more substituents, or an arylene group optionally having one or more substituents; and
wherein the content of each of Al, Cr, Cu, Fe, Ni, and Zn is 1000 ppm or less.

19. A compound represented by formula (5A): 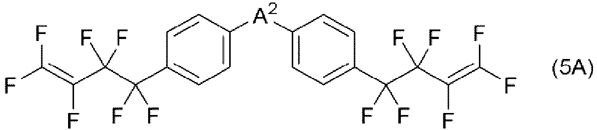
wherein A² is a single bond, -O-, -S-, an alkylene group optionally having one or more substituents, a cycloalkylene group optionally having one or more substituents, or an arylene group optionally having one or more substituents; and
wherein the total content of Al, Cr, Cu, Fe, Ni, and Zn is 6000 ppm or less.

20. A compound represented by formula (5): 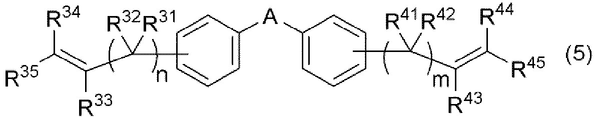
wherein A is a single bond, -O-, -S-, an alkylene group optionally having one or more substituents, a cycloalkylene group optionally having one or more substituents, or an arylene group optionally having one or more substituents,
R³¹, R³², R³³, R³⁴, R³⁵, R⁴¹, R⁴², R⁴³, R⁴⁴, and R⁴⁵ are each independently fluorine, an alkyl group, or a fluoroalkyl group, and
n and m are each independently 0, 1, or 2; and
wherein the Pd content is 1000 ppm or less.
